# EUROPÄISCHE PATENTSCHRIFT

(11) **EP 0 759 553 B1**
(45) Veröffentlichungstag und Bekanntmachung des Hinweises auf die Patenterteilung: **22.05.2002**
(21) Anmeldenummer: 96113118.2
(22) Anmeldetag: 15.08.1996
(51) Int. Cl.: G01N 27/416, G01N 27/30

(54) **Amperometrischer Biosensor**
Amperometric biosensor
Biocapteur ampérométrique

(30) Priorität: 18.08.1995 DE 19530376
(43) Veröffentlichungstag der Anmeldung: 26.02.1997
(73) Patentinhaber: Fresenius Medical Care Deutschland GmbH, 61352 Bad Homburg v.d.H. (DE)
(72) Erfinder: Abel, Petra, 61169 Friedberg (DE); Allendörfer, Wolfgang, 61352 Bad Homburg (DE)
(74) Vertreter: Luderschmidt, Schüler & Partner GbR

(56) Entgegenhaltungen:
- EP-A- 0 247 850
- EP-A- 0 447 288
- US-A- 4 950 379
- US-A- 5 227 042
- US-A- 5 269 891
- US-A- 5 286 364
- PATENT ABSTRACTS OF JAPAN vol. 013, no. 537 (P-968), 30.November 1989 & JP-A-01 221653 (SHIMADZU CORP), 5.September 1989,

## Beschreibung

Die Erfindung betrifft einen Biosensor zur amperometrischen Bestimmung eines in einer wässrigen Lösung, insbesondere Blut, gelösten Substrats mit einem Enuym zur Umsetzung des Substrats, einer Meßelektrode, deren Oberfläche sich für eine Redoxreaktion der Substratumsetzungsprodukte eignet, und die einen elektrisch leitenden Träger aus Kohlenstoff und ein auf den elekrisch leitenden Träger aufgebrachtes Metall der 8. Nebengruppe aufweist, wobei der elektrisch leitende Träger mit einer das Enzym enthaltenden Lösung getränkt ist, einer semipermeablen Membran, die die Meßelektrode einschließlich des elektrisch leitenden Trägers dicht umschließt, und einem Ableitkontakt, der von dem elektrisch leitenden Träger abgeht.

Biosensoren dienen zur Bestimmung eines Substrats, das mit Hilfe des im Biosensor vorgesehenen Enzyms in ein Umsetzungsprodukt katalytisch umgesetzt wird, das der Biosensor qualitativ und quantitativ bestimmen kann. Solche Enzymelektroden werden üblicherweise zur Bestimmung der Glukose in Blut eingesetzt, bei denen als Enzym Glukoseoxidase eingesetzt wird, mit deren Hilfe Glukose katalytisch in Glukonolakton/Glukonsäure und H₂O₂ umgesetzt wird. Bei der Amperometrie wird dann das Wasserstoffperoxid oxidiert nach folgender Gleichung:

H₂O₂ - 2H⁺+2e⁻+O₂.

Die an der Elektrode freigesetzten Elektronen werden als Oxidationsstrom abgeführt und sind in einem bestimmten Bereich proportional zur Glukosekonzentration.

Das vorstehende genannte Glukose- Biosensorsystem läßt sich ohne weiteres auf andere Systeme übertragen, beispielsweise auf das System Alkohol/Alkoholoxidase, Laktat/ Laktatoxidase, Harnsäure/ Urikase, Wasserstoffperoxid/Katalase und dergleichen.

Die üblicherweise eingesetzten Arbeitselektroden, die ein Enzym als Katalysator für die Umsetzung eines in Wasser gelösten Substrats aufweisen, arbeiten bei Referenzspannungen von 600 mV und mehr, was jedoch bei sämtlichen Meßsystemen bisher zu unerwünschten Korrosionen und anderen unerwünschten Nebenreaktionen geführt hat. Insofern wurde bereits versucht, diese Referenzspannung herabzusetzen, indem das Substratzersetzungsprodukt ebenfalls mit Hilfe eines Katalysators in dessen Folgeprodukt umgewandelt wird, wie dies vorstehend in der Umsetzungsgleichung gezeigt ist.

In der GB-PS 2,191,003 (EP 0 247 850) ist ein Biosensor in Form einer Enzymelektrode beschrieben, die in der Arbeitselektrode ein Metall der Platingruppe einsetzt. Um eine möglichst große Umsetzungsfläche zu erreichen, ist das Platinmetall in kolloider Form einheitlich verteilt auf einem elektrisch leitenden Träger vorgesehen. Dieses Trägermaterial liegt ebenfalls fein verteilt vor, üblicherweise als Kohlenstoffpulver, das mit Hilfe eines wasserabweisenden Bindemittelharzes verfestigt ist.

Eine derartig hergestellte Arbeitselektrode verfügt über hohe Stromdichten pro Flächeneinheit und weist üblicherweise eine Betriebsspannung von 300 - 350 mV gegenüber den vorstehend genannten Spannungen von 600 - 700 mV auf.

Auf dieser porösen Arbeitselektrode ist das Enzym entweder adsorbiert oder aber kovalent gebunden vorgesehen, wobei die Frontfläche der Arbeitselektrode mit einer porösen Membran abgedeckt sein kann, die gegenüber den zu bestimmenden Enzymsubstrat durchlässig ist.

Von dieser bekannten Arbeitselektrode gehen elektrische Kontakte, wie Platin, Silber und dergleichen ab.
Es hat sich nun herausgestellt, daß selbst ein elektrischer Kontakt aus dem üblicherweise eingesetzten Platin dem Biosensor keine lange Lebensdauer (maximal 1 Monat) garantiert, da er nach Ablauf dieser Periode korrodiert ist, so daß die gesamte Elektrode ausgetauscht werden muß. Auch andere Materialien, wie Gold oder Kupfer, haben sich bei dieser Elektrode als nicht einsetzbar erwiesen, da sie innerhalb weniger Wochen korrodiert waren.

Ähnliche Elektrodenanordnung sind in der EP- 470 290, EP-127 958, EP- 197 747, EP-359 831, US 4 655 880, US 4 950 379, PCT-WO 89/05454, DD 297 713, US 52 27 042, EP-A- 247 850 oder JP-A- 12 21 653 beschrieben.

So weist die Arbeitselektrode gemäß EP 0 470 290 Glaskohlenstoff als Sensormaterial auf, das unmittelbar mit der Enzymschicht verbunden ist. Da es sich hier um keinen katalytisch wirksamen Sensor handelt, treten unterhalb einer Arbeitsspannung von 600 mV keine Effekte bei einer Glukose/ Glukoseoxidase - Elektrodenanordnung auf, so daß auch dort die eingangs erwähnten unerwünschten Spannungen auftreten. US 5 227 042 beschreibt eine Kohlenstoffslektrode aus Glaskohlenstoff in dem eine Kunststofffolie einpolymerisiert wird. Die Erfindung liegt daher die Aufgabe zugrunde, die eingangs erwähnte Elektrodenanordnung so zu verbessern, daß sie bei möglichst niedriger Arbeitsspannung, d. h. unterhalb 500 mV arbeitet und darüber hinaus langzeitstabil ist, also keine Korrosionseffekte zeigt.

Die Lösung der Aufgabe gelingt dadurch, daß der Ableitkontakt aus Glaskohlenstoff besteht, wobei der elektrisch leitende Träger mit einer das Enzym enthaltenden Lösung getränkt ist und eine semipermeable Membran aufweist, die die Meßelektrode einschließlich des elektrisch leitenden Trägers dicht umschließt.
Der Einsatz von Glaskohlenstoff, der mit der Arbeitselektrode elektrisch- leitend verbunden ist, hat zunächst den Vorteil, daß die Elektrode keinerlei Korrosionserscheinungen während der Lebensdauer der Elektrode unterzogen wird, die praktisch nur durch die Enzymaktivität bestimmt wird. Die Lebensdauer des Enzyms liegt üblicherweise bei 4 - 6 Monaten, also einer Zeitperiode, bei der sich keinerlei korrosive Veränderungen an der Glaskohlenstoffoberfläche zeigen. Es treten nur minimale oder gar keine Störpotentiale oder -spannungen an der Elektrode auf, so daß das gesamte Meßverfahren hierdurch erheblich vereinfacht wird. Dies führt dazu, daß mit der erfindungsgemäßen Elektrode direkt Messungen in unverdünnten Vollblut möglich sind, was unter anderem an Dauermessungen mit Humanblut nachvollzogen worden ist. Neben Vollblut können jedoch auch andere wässerige Körperflüssigkeiten, wie Serum, Plasma, Urin, Speichel, Dialyseflüssigkeiten, Elektrolytlösungen und dergleichen mehr auf das zu untersuchende Substrat, das in einer derartigen Lösung enthalten ist, mit dem erfindungsgemäßen Sensor untersucht worden.

Der erfindungsgemäße Biosensor wird vorteilhafterweise zur Bestimmung von Glukose eingesetzt, wobei als Enzym in diesem Fall Glukoseoxidase (GOD) zum Einsatz kommt. Jedoch können auch andere Oxidoreduktasen eingesetzt werden, zu denen beispielsweise Laktatoxidase, Cholesterinoxidase, Galaktoseoxidase sowie andere Peroxid produzierende Enzyme und Kombinationen solcher Enzyme gehören. Auf die bereits vorstehend erwähnten Substrat/Oxidasesysteme wird ebenfalls Bezug genommen (Harnsäure/ Urikase; Ascorbinsäure/ Ascorbatoxidase; Pyruvat/Pyruvatoxidase).

Die einsetzbaren Enzyme können entweder an dem elektrisch leitenden Trägermaterial adsorbiert werden oder aber unmittelbar mittels einer chemischen Reaktion kovalent an diesen Träger gebunden, d. h. immobilisiert werden.

Was zunächst den Träger selbst betrifft, so wird auf die vorstehend genannte EP 0 247 850 aus Offenbarungsgründen Bezug genommen, deren Inhalt zum Gegenstand dieser Anmeldung gemacht wird.

Das erfindungsgemäß eingesetzte Trägermaterial besteht aus einer porösen Schicht kohlenstoffhaltiger Partikel, die untereinander mit einem Harz-Bindemittel gebunden sind. Die Größe dieser Partikel beträgt bis zu 50 nm.

Die Partikel selbst weisen ein Kohlenstoff- oder Graphitpulver auf, das eine hohe Dichte funktioneller Gruppen (Carboxylate, amino- und schwefelenthaltende Gruppen) auf der Oberfläche aufweisen kann. Dieses Pulver kann auf Grund seiner großen Oberfläche sehr leicht die vorstehend genannten eingesetzten Enzyme binden.

Auf diesem Pulver wird vorteilhafterweise vor dessen Kompatieren mit Hilfe eines Bindemittels das Metall der 8. Nebengruppe in kolloider Suspension bis zu 20 Gewichtsprozent bezogen auf den Kohlenstoffträger aufgetragen, so daß sich eine einheitliche Verteilung des Platins oder Palladiums als Metall der Platingruppe vorteilhafterweise in den Kohlenstoffträger ergibt. Nach dem Vermischen mit dem platinhaltigen Material wird der Kohlenstoffträger mit einem üblicherweise wasserabstoßenden Harzbindemittel vermischt und in eine vorbestimmte Form überführt. Vorteilhafterweise werden fluorhaltige Harze, beispielsweise auf der Basis von PTFE als harzhaltige Bindemittel eingesetzt, wie dies in der vorstehend genannten EP-Schrift erläutert ist, worauf Bezug genommen wird. Dieses Bindemittel liegt in einer Menge bis zu 70 Gewichtsprozent vor, wobei dessen Gewichtsanteil üblicherweise nicht kritisch ist.

Das Bindemittel soll dabei eine minimale Sauerstoffdurchlässigkeit unter atmosphärischen Bedingungen, wenigstens 2 x 10⁻³ cm³ O₂/ cm³ bezogen auf das Polymer, haben.

Desweiteren soll die Partikelgröße des kolloidalen Platin-Metalls in einem Bereich von etwa 1 - 3 nm liegen, das an die Oberfläche des Kohlenstoffpulver adsorbiert ist.

Vorteilhafterweise kann das elektrisch leitende Trägergemisch mit dem Platinmetall in eine Folie geformt werden, die vorteilhafterweise auf einer Kohlenstoffolie als Trägermaterial fixiert ist.

Bevorzugte Enzymelektrodensubstrate werden unter der Bezeichnung PACE von der Firma E-TEK vertrieben und werden üblicherweise als elektro-katalytische Gasdiffusionselektroden in Brennstoffzellen eingesetzt.

Wie vorstehend erwähnt, kann das Enzym am Träger adsorbiert oder aber unmittelbar immobilisiert werden. Erfindungsgemäß ist die Adsorption eines Enzyms dann bevorzugt, wenn die Oberfläche des so behandelten Trägers mit einer mikroporösen, semipermeablen Membran gegenüber dem wässerigen Meßgut geschützt ist. Andererseits kann natürlich das Enzym durch eine Immobilisierungsbehandlung physikalischer oder chemischer Natur so fest an dem Träger haften, daß ein derartiger Schutz durch eine Membran nicht notwendig ist.

Bei der reinen Adsorption wird das Enzym in einer wässerigen Lösung oder Suspension vorgelegt, wobei diese Lösung auf den porösen Träger aufgetragen wird. Eine derart mit Enzym beschichtete Folie wird dann mit dem nachstehend erläuterten Ableitkontakt verbunden.

Andererseits kann das Enzym auf der Oberfläche des Trägers nach bekannten Immobilisierungstechniken, beispielsweise durch kovalente Bindung mit Carbodiimid oder Glutaraldehyd verbunden werden, wie dies in der vorstehenden EP-Schrift erläutert ist, worauf wiederum Bezug genommen wird.

Der elektrische Ableitkörper oder -kontakt besteht aus Glaskohlenstoff, der durch Pyrolyse von Polymeren mit dreidimensionaler vemetzter Struktur gebildet wird. Im Makrobereich hat glashaltiger Kohlenstoff praktisch keine Poren, besitzt jedoch in seinen Schichten zahlreich Hohlräume. Er ist außerordentlich korrosionsbeständig gegen Säuren und Alkalien sowie Schmelzen und wird erst oberhalb von etwa 550°C durch Sauerstoff bzw. oxidierende Schmelzen angegriffen.

Weitere Einzelheiten zu Glaskohlenstoff sind in der Zeitschrift für Werkstofftechnik 15 (1984) Seite 331 - 338 beschrieben, worauf bezug genommen wird. Erfindungsgemäß einsetzbare Glaskohlenstoffe werden in Form von platt-, ring-, stäbchen- und scheibenförmigen Elektroden für die chemische Analytik vertrieben. Darüberhinaus läßt sich die Oberfläche des Glaskohlenstoffs mechanisch bearbeiten, beispielsweise lassen sich Ringnuten und Bohrungen in zylindrische Körper einarbeiten.

Sofern erwünscht, kann die Oberflächenstruktur von Glaskohlenstoff durch Behandlung bei erhöhten Temperaturen, beispielsweise etwa 500°C, oder chemisch durch Einwirken von Salpetersäure aktiviert werden.

Erfindungsgemäß wird speziell für die Gegenelektrode im erfindungsgemäßen Meßverfahren aktivierte Glaskohlenstoff eingesetzt, während der Ableitkontakt der Arbeitselektrode aus üblicherweise nicht aktiviertem Glaskohlenstoff besteht.

Bei der Arbeitselektrode wird als Ableitkontakt ein stäbchenförmiger oder zylinderförmiger Körper eingesetzt, dessen Frontteil als Träger für den elektrisch leitenden Träger der Platinmetallelektrode dient. Sie nimmt weiterhin an ihrem rückwärtigen Ende einen Ableitdraht auf, der vorteilhafterweise in eine Axialbohrung des Glaskohlenstoffkörpers festsitzend und elektrisch leitend eingeführt ist.

Die erfindungsgemäße Arbeitselektrode wird vorteilhafterweise in Durchflußmeßzellen in Form einer 2- oder 3-Elektrodenanordnung eingesetzt.

Bei der 2-Elektrodenanordnung dient die Gegenelektrode zugleich auch als Bezugselektrode, während bei der 3-Elektrodenanordnung neben der Gegenelektrode eine Bezugselektrode vorliegt.

Bevorzugt ist bei der erfindungsgemäßen Meßzelle eine 3-Elektroden- anordnung, die neben der erfindungsgemäßen Meßelektrode eine Ag/AgCl-Elektrode als Bezugselektrode und eine Gegenelektrode aus aktiviertem Glaskohlenstoff enthält.

Desweiteren können Sensoren für die Temperaturmessung oder weitere Elektroden zur Korrektur von Störstoffen, wie Rinderserumalbumin - Hilfselektroden eingesetzt werden.

Die Beispiele erläutern die Erfindung.

Es zeigen
- Figur 1: die Seitenansicht eines Glaskohlenstoffstiftes der Arbeitselektrode, teilweise aufgerissen, im Bereich des Kontaktstiftes;
- Figur 2: eine Meßzelle mit 3 Elektroden, wobei die Arbeitselektrode und Meßelektrode im Ausschnitt und im Aufriß gezeigt sind;
- Figur 3: eine vergrößerte Darstellung des Detail A von Figur 2, wobei die Meßelektrode und die Referenzelektrode geschnitten dargestellt sind; und
- Figur 4: einen Schnitt durch die Meßzelle von Figur 2 entlang der Linie IV-IV, wobei lediglich die Gegenelektrode eingesetzt ist.

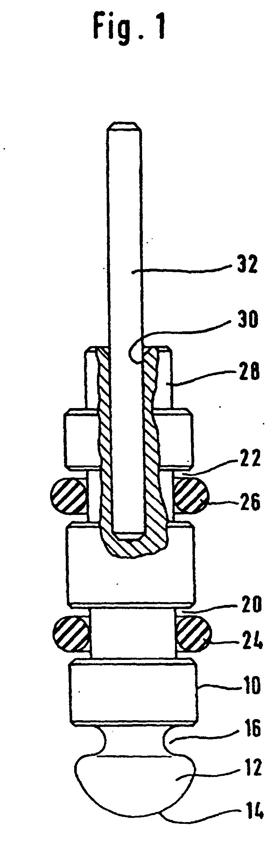

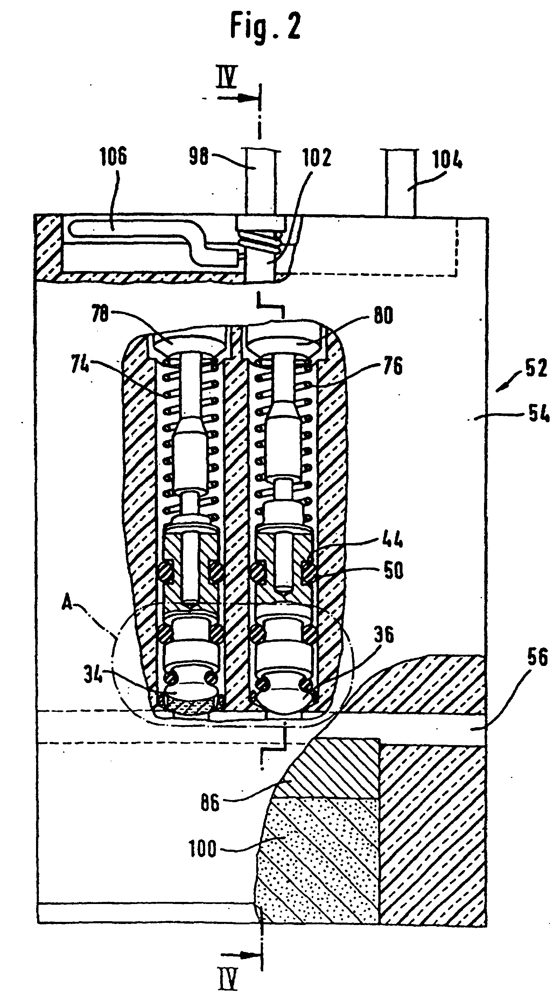

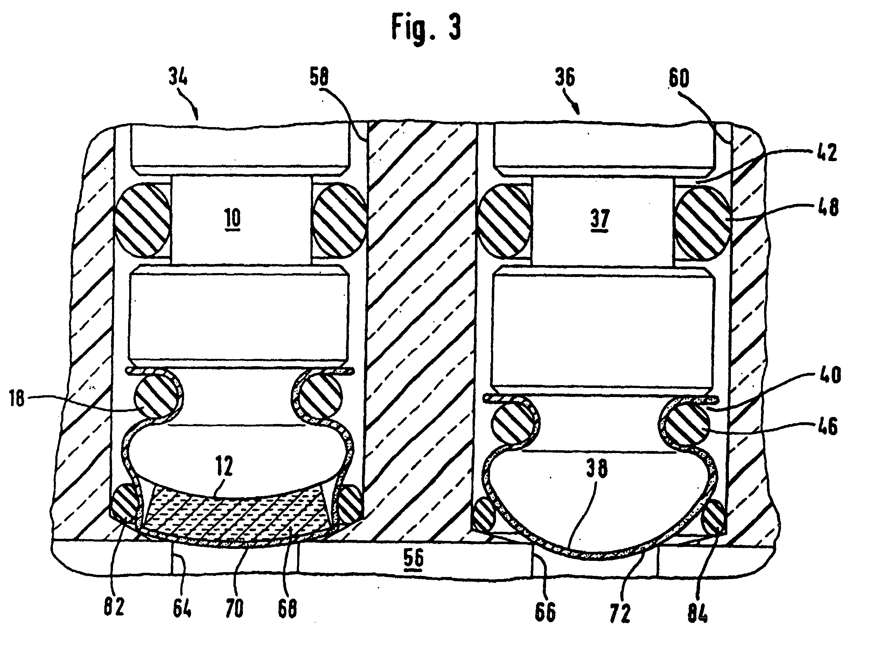

Aus Figur 1 ist der Elektrodenkörper 10 aus Glaskohlenstoff ersichtlich, der im wesentlichen eine zylinderförmige Struktur aufweist. Das Frontteil 12 des Elektrodenkörpers 10 ist gemäß der in Figur 1 gezeigten Ausführungsform nach außen gewölbt ausgebildet, was - wie speziell bei der Meßzelle gemäß Figur 2 nachstehend erläutert wird - die Anströmung der zu untersuchenden Lösung begünstigt. Benachbart zu dieser Wölbung 14 ist eine erste Ringnut 16 im Elektrodenkörper vorgesehen, in die ein erster O-Ring 18, wie aus Figur 3 ersichtlich ist, eingelegt werden kann.

Im Anschluß an diese erste Ringnut sind weitere Ringnuten 20 und 22 über den Elektrodenkörper verteilt vorgesehen, in die weitere O-Ringe 24 und 26 eingelegt werden können.

Am rückwärtigen Ende des zylinderförmigen Elektrodenkörpers 10 ist vorteilhafterweise ein zylinderförmiger Absatz 28 vorgesehen, der gegenüber dem Elektrodenkörper 10 einen geringeren Durchmesser aufweist.

Desweiteren ist von der Rückseite her eine axiale Bohrung 30 im Elektrodenkörper 10 vorgesehen, innerhalb der ein Kontaktstift 32 elektrisch leitend vorgesehen ist, beispielsweise mit Hilfe eines elektrisch leitenden Klebers.

Wie ebenfalls auf Figur 3 ersichtlich ist, unterscheiden sich die Elektrodenkörper 10 der Meßelektrode 34 und der Bezugselektrode 36 in ihrer Struktur nur dadurch, daß die Wölbung der Bezugselektrode 36 in ihrem Frontbereich 38 stärker ausgebildet ist.

Die Referenzelektrode besteht jedoch nicht - wie vorstehend erwähnt - aus Kohlenstoff, sondern aus einem Silber/Silberchlorid-Stift, der die entsprechenden Ringnuten 40 - 44 und O-Ringe 46 - 50 aufweist.

Gemäß Figuren 2 und 3 ist eine Meßzelle 52 dargestellt, die aus einem Elektrodenblock 54, üblicherweise aus einem transparenten Kunststoffmaterial, wie Acrylglas, besteht, der einen Meßkanal 56 aufweist, der sich quer durch den Elektrodenblock 54 erstreckt. Senkrecht von oben sind erste, zweite und dritte Bohrungen im Elektrodenblock 54 vorgesehen, von denen die erste und die zweite Bohrung 58, 60 bis zum Meßkanal 56 geführt sind, wobei die Durchgangsöffnung 64 bzw. 66 der ersten Bohrung 58 bzw. der zweiten Bohrung 60 zum Meßkanal 56 hin gegenüber dem Durchmesser dieser beiden Bohrungen 58 und 60 verengt ist, d. h. einen geringeren Durchmesser im Vergleich zum Bohrungsdurchmesser aufweist.

Dabei ist der Durchmesser der Bohrungen 58 und 60 so dimensioniert, daß dieser geringfügig größer ist als der Durchmesser des Elektrodenkörpers 10 der Meßelektrode 34 bzw. der Bezugselektrode 36. Andererseits sind jedoch die Außendurchmesser der O-Ringe 24 und 26 bzw. 48 und 50 etwas größer als der Durchmesser der Bohrungen 58 und 60, so daß es hier zu einer radialen Dichtung zwischen O-Ring/Bohrungswand kommt.

Wie weiterhin aus Figur 3 ersichtlich ist, ist auf der Frontseite 12 der Arbeitsoder Meßelektrode 34 der vorstehend erwähnte elektrisch leitende Träger 68 mit einem Platinmetall vorgesehen, der weiterhin mit einer Enzym-Lösung getränkt ist. Soll Glukose, beispielsweise im Blut mit der Meßelektrode 34 bestimmt werden, so ist als Enzym Glukoseoxidase in dem Träger 68 vorgesehen, der aus einer mit kolloidalem Platin aktivierten Kohlenstoffolie besteht.

Um den Frontbereich 12 des Elektrodenkörpers 10 und den auf dem Frontbereich vorgesehenen Pace-Träger 68 ist eine semipermeable Membran 70 umhüllend angeordnet, die sich nach rückwärts bis über die erste Ringnut 16 hinaus erstreckt und vom O-Ring 18 in der Ringnut dicht gegenüber der Umgebung fixiert ist. Die in der Pace-Folie 68 vorgesehene Enzym-Lösung befindet sich innerhalb der Membran 70. Insofern ist die Glukoselösung in dem von der Membran 70 durch die Wirkung des O-Rings 18 gebildeten Raums dicht eingeschlossen und somit vor Einfüssen der Umgebung, insbesondere des zu messenden Guts, geschützt. Gleiches gilt für die Bezugselektrode 36, deren Frontbereich 38 ebenfalls von einer Membran 72 durch die Wirkung des O-Rings 46 geschützt ist.

Gemäß Figur 2 ist der Einbau der Meßelektrode 34 und 36 in den Elektrodenblock 54 ersichtlich. Wie bereits vorstehend erläutert, werden die Elektroden radial mit Hilfe der O-Ringe 24, 26 bzw. 48 und 50 innerhalb der Bohrungen 58 bzw. 60 dicht angeordnet und außerdem geführt. Desweiteren erfolgt eine axiale Spannung der Elektroden 34 und 36 gegen die Durchtrittsöffnungen 64 und 66 mit Hilfe von Federn 74 bzw. 76, die auf den rückwärtigen Bereich des Elektrodenkörpers 10 bzw. 37 drücken, wobei der Absatz 28 als Führung für die Feder 74 dient. Die Spannung der Federn 74 und 76 innerhalb der Bohrungen 48 und 60 erfolgt dabei über hohle Stopfen oder Schrauben 78 bzw. 80, wie diese ebenfalls auf Figur 2 ersichtlich ist. Um eine Beschädigung des Frontbereichs 12 bzw. 38 und der diesen Frontbereich 12, 38 überziehenden Membran 70 und 72 zu verhindern, ist jeweils in die Bohrung 60 und 62 benachbart zur Durchtrittsöffnung 64 und 66 jeweils ein O-Ring 82 und 84 eingelegt, gegen den sich der Außenrand des Frontbereichs 12, 38 dichtend federnd legt, wobei die Wölbungen der Frontseiten 14 und 38 in den Meßkanal 56 hineinragen, so daß durch die so erzielten, günstigen Anströmverhältnisse ein optimaler Probenkontakt erzielt wird. Durch die Vorwölbung in den Probenkanal kommt es zu einer totzonenfreien Anordnung, so daß hierdurch eine besonders probenverschleppungsarme Pobenbehandlung gegeben ist.

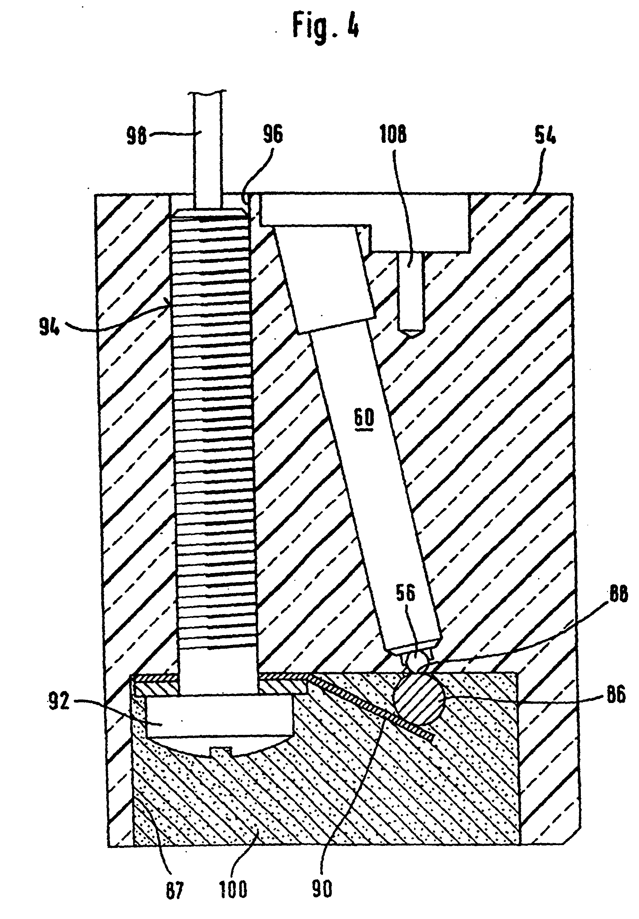

Gemäß Figur 4 ist lediglich die Gegenelektrode 86 dargestellt, die, wie in Verbindung mit der Darstellung von Figur 2 ersichtlich ist, zylinderförmig ausgebildet ist. Die Längsachse dieser Gegenelektrode 86 läuft paralell zur Längsachse des Meßkanals 56, der auf seiner Unterseite einen entsprechend langen Schlitz 88 aufweist, gegen den die Außenfläche der Gegenelektrode 86 mit Hilfe eines Federelements 90 gedrückt ist. Dieses Federelement ist mit einer Schraube 92 am Elektrodenblock 54 befestigt, in dem zum Zwecke der Befestigung eine senkrechte Bohrung 94 vorgesehen ist, innerhalb der die Schraube mittels eines in der Bohrung vorgesehenen Gewindes 96 befestigt ist. Sowohl das Federelement 90 als auch die Schraube 92 sind elektrisch leitend, wobei die Schraube 92 über einen Kontaktstift 98 nach außen an den Meßstromkreis angeschlossen werden kann.

Wie aus Figur 4 ersichtlich ist, wird Zu Montagezwecken die Gegenelektrode 86, das metallische Federelement 90 und Schraube 92 von der Unterseite des Elektrodenblocks 54 her in eine im wesentlichen rechteckige Öffnung 87 eingeführt, die nach der Montage mit einem Gießharz 100 verschlossen wird. Die Gegenelektrode 86 selbst besteht - wie vorstehend erläutert - vorteilhaft aus aktiviertem Glaskohlenstoff.

Wie aus Figur 2 weiterhin ersichtlich ist, ragen aus dem Elektrodenblock 2 weitere Kontaktstifte 102 und 104 heraus, die über elektrische Leitungen, von denen in Figur 2 nur die Leitung 106 (Meßelektrodenleitung) gezeigt ist, mit der Meßelektrode 34 bzw. der Bezugselektrode 36 verbunden sind. Die entsprechende Bohrung 108 für den Elektrodenstift 102 ist aus Figur 4 ersichtlich.

### Beispiel 1

Eine mit kolloidalem Platin aktivierte Kohlenstoffolie wird mit einer Glukoseoxidase-Lösung deart betropft, daß die Folie etwa einen Glukosoxidasegehalt von etwa 10 Enzymeinheiten je mm2 aufweist. Anschließend wird diese Folie in der Elektrodenanordnung gemäß Figur 2 - 4 verwendet.

Diese Folie wird mit einer hydrophilen semipermeablen Membran umhüllt, wobei die Membran einen mittleren Porendurchmesser von etwa 30 nm besitzt. Durch den Meßkanal 56 werden unterschiedliche Glukose-Konzentrationen in Wasser/Blut geführt, wobei sich folgende Strom-Spannungs-Kurve (Voltamogramm) ergibt. Der Diffusionsgrenzstrom liegt dabei für eine Glukosekonzentration von 20 mmol/l bei etwa 3 .A. Es treten nur minimale oder keine Störpotentiale/Polarisation auf. Desgleichen wird auch während der mittleren Lebensdauer der Elektrode (etwa 6 Monate), die nur durch den Verbrauch des Enzyms bestimmt ist, keine Korrosion beobachtet. Auf Grund der minimalen Probenverschleppung und des optimalen Probenkontakts treten deutliche, störungsfreie Signale auf. Die Meßwerte sind genauer und reproduzierbarer als bei den bisher eingesetzten Elektroden (2-Elektrodensystem vom Ag/Pt-Typ). Desgleichen ist die Linearität der Meßsignale bei unterschiedlichen Meßkonzentrationen verbessert und der Meßbereich selbst erweitert.

### Vergleichsversuch 1

Anstelle von Glaskohlenstoff wird in der Meßelektrode Platin als Elektrodenkörper 10 zur Stromableitung benutzt.

Schon nach kurzer Zeit treten Korrosionseffekte durch die üblicherweise zusätzlich auftretenden Batteriepotentiale auf.

### Vergleichsversuch 2

Anstelle der platinierten Kohlenstoff-Folie (Pace) wird eine nur aus Kohlenstoff bestehende Folie, die also nicht platiniert ist, eingesetzt. Ansonsten wird wiederum das Beispiel 1 wiederholt. Gegenüber den bei der Pace-Folie eintretenden Meßeffekten, zu deren Erzeugung ein Potential von etwa 330 mV anzulegen ist, tritt hier erst ein Meßeffekt bei etwa 845 mV auf, dabei ist die Messung begleitet von Elektrolyse-Störeffekten, so daß diese Elektrode nur bedingt eingesetzt werden kann.

## Patentansprüche

1. Biosensor zur amperometrischen Bestimmung eines in einer wässerigen Lösung, insbesondere Blut, gelösten Substrats mit einem Enzym zur Umsetzung des Substrats, einer Meßelektrode (34), deren Oberfläche sich für eine Redoxreaktion der Substratumsetzungsprodukte eignet, und die einen elektrisch leitenden Träger aus Kohlenstoff (68), ein auf den elektrisch leitenden Träger aufgebrachtes Metall der 8. Nebengruppe und einen Ableitkontakt (10) aufweist, der von dem elektrisch leitenden Träger (68) abgeht, **dadurch gekennzeichnet, daß** der Ableitkontakt (10) aus Glaskohlenstoff besteht, wobei der elektrisch leitende Träger mit einer das Enzym enthaltenden Lösung getränkt ist und eine semipermeable Membran aufweist, die die Meßelektrode einschließlich des elektrisch leitenden Trägers dicht umschließt.

2. Biosensor nach Anspruch 1 **dadurch gekennzeichnet, daß** der Glaskohlenstoff aktiviert ist.

3. Biosensor nach Anspruch 1 oder 2, **dadurch gekennzeichnet, daß** der Ableitkontakt (10) ein Zylinderteil aufweist, auf dessen Frontseite die Arbeitselektrode angeordnet ist.

4. Biosensor nach einem der Ansprüche 1 - 3, **dadurch gekennzeichnet, daß** die Frontseite (12) nach außen gewölbt ist.

5. Biosensor nach einem der Ansprüche 1 - 4, **dadurch gekennzeichnet, daß** der zylinderförmige Elektrodenkörper auf seiner Oberfläche eine erste Ringnut (16) zur Aufnahme eines O-Rings (18) aufweist, mit dem die Membran (70) am Elektrodenkörper (10) fixierbar ist.

6. Bionsensor nach einem der Ansprüche 1 - 3, **dadurch gekennzeichnet, daß** der zylinderförmige Elektrodenkörper weitere Ringnuten (20, 22) zur Aufnahme von weiteren O-Ringen (24, 26) aufweist, mit denen der Elektrodenkörper (10) gegen eine in einem Elektrodenblock (54) vorgesehene Bohrung (58) radial fixierbar ist.

7. Meßzelle (52), aufweisend den Biosensor nach Anspruch 1, eine Gegenelektrode und eine Bezugselektrode.

8. Meßzelle nach Anspruch 7, **dadurch gekennzeichnet, daß** sie einen Meßkanal (56), eine erste Bohrung 58, in dem die Meßelektrode (34) gemäß Anspruch 1 angeordnet ist, und eine zweite Bohrung (60) aufweist, in der die Bezugselektrode (36) angeordnet ist, wobei die erste und zweite Bohrung (58) und (60) im wesentlichen senkrecht zum Meßkanal (56) angeordnet sind und in diesen münden, und die Gegenelektrode (86) zumindest einen Teil der Wand des Meßkanals (56) bildet.

9. Meßzelle nach Anspruch 7 oder 8, **dadurch gekennzeichnet, daß** die Gegenelektrode (86) aus Glaskohlenstoff besteht, der vorzugsweise aktiviert ist, und zylinderförmig ausgebildet ist.

10. Meßzelle nach Anspruch 7 oder 8 **gekennzeichnet durch** eine Bezugselektrode (36) aus Silber/Silberchlorid.

11. Meßzelle nach einem der Ansprüche 7 bis 9, **dadurch gekennzeichnet, daß** die Längsachse der Gegenelektrode (86) zur Längsachse des Meßkanals (56) im wesentlichen parallel ist und die Gegenelektrode (86) gegen einen am Meßkanal (56) angeordneten Schlitz (88), der im Elektrodenblock (54) vorgesehen ist, gedrückt ist.

## Claims

1. Biosensor for amperometric determination of a substrate dissolved in an aqueous solution, particularly blood, with an enzyme for converting the substrate, a measuring electrode (34) whose surface is suitable for a redox reaction of the substrate conversion products, and which exhibits an electrically conducting carbon support (68), a metal of the 8^{th} secondary group applied to the electrically conducting support, and a discharge contact (10) which departs from the electrically conducting support (68), **characterised in that** the discharge contact (10) consists of glass carbon, the electrically conducting support being saturated with a solution containing the enzyme and exhibiting a semi-permeable membrane which tightly encloses the measuring electrode, including the electrically conducting support.

2. Biosensor according to claim 1, **characterised in that** the glass carbon is activated.

3. Biosensor according to claim 1 or 2, **characterised in that** the discharge contact (10) exhibits a cylindrical section on the front of which is arranged the working electrode.

4. Biosensor according to one of claims 1 - 3, **characterised in that** the front (12) is curved outwards.

5. Biosensor according to one of claims 1 - 4, **characterised in that** the cylindrical electrode body exhibits on its surface a first annular groove (16) for receiving an O ring (18) with which the membrane (70) can be secured to the electrode body (10).

6. Biosensor according to one of claims 1 - 3, **characterised in that** the cylindrical electrode body exhibits further annular grooves (20, 22) for receiving further O rings (24, 26) with which the electrode body (10) can be secured radially against a hole provided in the electrode block (54).

7. Measuring cell (52) exhibiting the biosensor according to claim 1, a counter-electrode and a reference electrode.

8. Measuring cell according to claim 7, **characterised in that** it exhibits a measuring channel (56), a first hole 58, in which the measuring electrode (45) according to claim 1 is arranged, and a second hole (60) in which the reference electrode (36) is arranged, the first and second holes (58) and (60) being arranged essentially perpendicular to the measuring channel (56), and opening into it, and the counter-electrode (86) forming at least part of the wall of the measuring channel (56).

9. Measuring cell according to claim 7 or 8, **characterised in that** the counter-electrode (86) consists of glass carbon which is preferably activated, and is designed in the shape of a cylinder.

10. Measuring cell according to claim 7 or 8 **characterised by** a reference electrode (36) of silver/silver chloride.

11. Measuring cell according to one of claims 7 to 9, **characterised in that** the longitudinal axis of the counter-electrode (86) is essentially parallel with the longitudinal axis of the measuring channel (56), and **in that** the counter-electrode (86) is pressed against a slot (88) arranged on the measuring channel (56), which slot is provided in the electrode block (54).

## Revendications

1. Biocapteur pour la détermination ampérométrique d'un substrat dissous dans une solution aqueuse, en particulier du sang, au moyen d'un enzyme de conversion du substrat, d'une électrode de mesure (34), dont la surface convient à une réaction REDOX des produits de conversion du substrat, et qui présente un support conducteur électrique en carbone (68), un métal du sous-groupe 8 déposé sur le support conducteur électrique et un contact de dérivation (10), partant du support conducteur électrique (68), **caractérisé en ce que** le contact de dérivation (10) est constitué de composite carbone verre, tandis que le support conducteur électrique est imbibé d'une solution contenant l'enzyme et présente une membrane semi-perméable qui enferme hermétiquement l'électrode de mesure y compris le support conducteur électrique.

2. Biocapteur selon la revendication 1, **caractérisé en ce que** le composite carbone verre est activé.

3. Biocapteur selon la revendication 1 ou 2, **caractérisé en ce que** le contact de dérivation (10) présente une partie cylindrique, à la face frontale de laquelle est disposée l'électrode de travail.

4. Biocapteur selon une des revendications 1 à 3, **caractérisé en ce que** la face frontale (12) est bombée vers l'extérieur.

5. Biocapteur selon une des revendications 1 à 4, **caractérisé en ce que** le corps d'électrode cylindrique présente sur sa surface une première gorge annulaire (16) pour loger un joint torique (18), avec lequel la membrane (70) peut être fixée sur le corps d'électrode (10).

6. Biocapteur selon une des revendications 1 à 3, **caractérisé en ce que** le corps d'électrode cylindrique présente d'autres gorges annulaires (20, 22) pour loger d'autres joints toriques (24, 26), avec lesquels le corps d'électrode (10) peut être fixé radialement par rapport à un trou (58) prévu dans un bloc électrode (54).

7. Cellule de mesure (52), présentant le biocapteur selon la revendication 1, une contre-électrode et une électrode de référence.

8. Cellule de mesure selon la revendication 7, **caractérisée en ce qu'**elle présente un canal de mesure (56), un premier trou (58), dans lequel est disposée l'électrode de mesure (34) selon la revendication 1, et un autre trou (60), dans lequel est disposée l'électrode de référence (36), le premier et le deuxième trou (58) et (60) étant disposés sensiblement perpendiculairement au canal de mesure (56) et débouchant dans ce dernier, et la contre-électrode (86) formant au moins une partie de la paroi du canal de mesure (56).

9. Cellule de mesure selon la revendication 7 ou 8, **caractérisée en ce que** la contre-électrode (86) est constituée de composite carbone verre, qui est de préférence activé, et façonnée en forme de cylindre.

10. Cellule de mesure selon la revendication 7 ou 8, **caractérisée par** une électrode de référence (36) en argent/chlorure d'argent.

11. Cellule de mesure selon une des revendications 7 à 9, **caractérisée en ce que** l'axe longitudinal de la contre-électrode (86) est sensiblement parallèle à l'axe longitudinal du canal de mesure (56) et que la contre-électrode (86) est appliquée contre une fente (88) disposée sur le canal de mesure (56), qui est prévue dans le bloc électrode (54).
